# EUROPEAN PATENT SPECIFICATION

(11) **EP 2 379 698 B1**
(45) Date of publication and mention of the grant of the patent: **21.11.2018**
(21) Application number: 09829870.6
(22) Date of filing: 18.12.2009
(51) Int. Cl.: C12M 3/00

(54) **APPARATUS AND METHOD FOR HANDLING FLUIDS FOR ANALYSIS**
VORRICHTUNG UND VERFAHREN ZUR HANDHABUNG VON FLÜSSIGKEITEN FÜR ANALYSEZWECKE
DISPOSITIF ET PROCÉDÉ DE MANIPULATION DE FLUIDES POUR ANALYSE

(30) Priority: 22.12.2008 US 139644 P
(43) Date of publication of application: 26.10.2011
(73) Proprietor: Abbott Laboratories, Des Plaines, IL 60018 (US); Sage, Russell L, McHenry, Illinois 60050 (US)
(72) Inventor: SAGE, Russell, L, McHenry, Illinois 60050 (US)
(74) Representative: Modiano, Micaela Nadia
(86) International application number: PCT/US2009/068651
(87) International publication number: WO 2010/075199

(56) References cited:
- EP-A2- 0 734 767
- WO-A2-2004/035210
- WO-A2-2008/073691
- DE-C1- 19 806 780
- GB-A- 2 139 519
- JP-A- 58 067 339
- US-A1- 2005 047 963

## Description

### TECHNICAL FIELD

The present invention is directed toward devices and methods for extracting a compound of interest from specimens, and particularly toward an apparatus and method for processing specimens during testing, including adding fluids such as reagents during the processing of specimens.

### BACKGROUND OF THE INVENTION AND TECHNICAL PROBLEMS POSED BY THE PRIOR ART

Extraction and analysis of analytes of interest (analytes), such as DNA, RNA, and proteins, from biological samples, or specimens, is important for the identification of many medical conditions. The specimens containing the analytes of interest include urine, blood, serum, plasma, cerebral-spinal and lymph fluids, and tissues. The analytes of interest, after extraction and purification, are consequently analyzed using a variety of techniques and instruments.

Extracting the compound in relatively pure form is imperative in producing an unequivocal indication of medical conditions in most tests. The extraction must also avoid cross contamination of specimens and/or analytes of interest. This is particularly relevant when the extracted analyte is a nucleic acid subjected to amplification techniques such as the polymerase chain reaction (PCR), which are particularly susceptible to contamination.

Extraction is commonly accomplished using automated devices that handle multiple specimens and reagents (typically liquid reagents). The automated devices typically use sets of pipettes to move various fluids between their original containers (usually receptacles such as open topped tubes) and containers in which the specimens are to be processed. For example, a set of 8 specimens may be contained in 8 tubes or other receptacles loaded in a rack on the device, and a head carrying 8 pipettes will, through programmed motion, move the pipettes into those 8 tubes, where a selected amount of each specimen will be aspirated from its tube into the pipettes. The head will then retract the pipettes from the tubes and move to another set of tubes located at a processing station, depositing the extracted amounts of each specimen from the pipettes into sets of testing tubes (reaction vessels).

Unless otherwise stated, the terms tube, testing tube and reaction vessel (RV) are used interchangeably herein.

Processing of the specimens and the reagents to accomplish extraction of the analyte of interest may additionally require heating to facilitate lysis of microorganisms or to maximize a specific chemical interaction.

In some processes, magnetic fields have been used to assist in separating analytes of interest from the fluid in the tubes. For example, certain analytes can be forced to bind to specific magnetic particles within a reagent. A magnetic field is then applied to pull the particles and the bound analyte to one side of the tube, whereby the reagent may be drawn out of the tube leaving the magnetic microparticle-analyte in a concentrated form.

Liquid handling devices utilize automation mechanisms to perform the extraction from multiple samples in order to increase the throughput (the number of tests performed per unit time) of the instrument and provide economy of scale. The extraction devices also provide for metering of reagents and for delivery of the reagents to the specimen tubes. Delivery is usually accomplished using pipetting mechanisms and disposable pipette tips. Generally, multiple pipette tips are used to deliver processing reagents to, and siphon unwanted reagents from, the testing tubes. The number of steps involving fluid exchange can be large, leading to high labor and commodities cost. Even for automated instruments, longer time is consumed in the loading, aspirating and dispensing of the pipette tips, which leads to longer turn around time. Excessive usage of pipette tips is a common complaint from cost and waste disposal issues, which the present invention minimizes by the introduction of innovative RVs with actuatable valves, where reagents can be drained from an opening in the bottom of the RVs, and methods for their use.

U.S. Pat. No. 6,117,398 alternatively discloses drawing fluid from the bottom of a sample vessel, wherein a valve is situated between every sample processing vessel and the waste container. U.S. Application No. 2005/0047963 A1 also discloses a reaction vessel with a bottom opening, with the emphasis on utilizing hydrophobic forces to prevent fluid flow through the bottom opening.

EP0734767 discloses a vessel, for treating liquids without contamination, with a liquid inlet opening and a liquid outlet opening, wherein the outlet is closed by a component which presses elastically against the opening and can be reversibly opened by reduced or elevated pressure, the component preferably exerts a permanent force in the direction of the outlet opening, the vessel is preferably hollow cylindrical and may have a slightly tapering outside shape with a precisely cylindrical interior.

### SUMMARY OF THE INVENTION

In one aspect of the present invention, a reaction vessel for the extraction of an analyte in a fluid is provided having:
i. a cylindrical body including a fluid containment space defined by substantially vertical walls, the cylindrical body including a conical thinned bottom portion
ii. an opening at the top of said vertical walls; and
iii. an elastic valve secured to the vertical walls to define the bottom of said containment space, the elastic valve including a conical thinned rim around its upper portion and a shoulder beneath the conical thinned rim, the conical thinned bottom portion of the cylindrical body is received in the conical thinned rim such that a bottom of said vertical walls abut said shoulder,
said elastic valve being concave and semispherical at the center of the containment space, said elastic valve having a slit positioned at a lowermost portion of the bottom of the containment space and being through the bottom of the concave center portion of the elastic valve, said slit having a closed condition blocking fluid from passing through said slit and an open position defining a drain opening allowing fluid to pass through said slit, and
said elastic valve adapted to
support a quantity of fluid in said containment space of said reaction vessel with said slit in said closed condition, and
be elastically deformed and drawn down to force said slit in said open position responsive to a selected differential between a first pressure at the top opening and a second pressure beneath the elastic valve, wherein the first pressure is greater than the second pressure,
wherein said elastic valve is constructed of a material possessing elastomeric properties such that said slit is normally in said closed condition, said lowermost portion of said bottom of said containment space flexing to open said slit to said open position upon application of said selected differential and said slit closing after the selected differential ceases.

In one form of the invention a lip extends downwardly beyond and around the concave bottom of the elastic valve. In another further form, the concave bottom of the elastic valve is semispherical.

In another form of the invention, the first pressure is atmospheric pressure and the second pressure is a partial vacuum.

In still another form of the invention, the closing walls are substantially cylindrical with a bottom portion tapered conically inwardly, and the conically tapered bottom portion is secured in an upper tapered portion of the elastic valve.

In yet another form of the invention, the elastic deformation comprises the concave center being drawn down.

The valve is a material which is inert with regard to interaction with reagents utilized in nucleic acid extraction in yet another form of the invention.

In another aspect of the invention, as defined in claim 7, a device for the extraction of an analyte in a fluid is provided.

In another aspect of the invention, as defined in claim 11, a method is provided for extracting an analyte in a fluid using reaction vessels according to the first aspect of the invention.

### BRIEF DESCRIPTION OF THE DRAWINGS

**FIG. *1a*** is a cross-sectional view of an RV which includes various aspects of the present invention.
**FIG. *1b*** is an enlarged cross-sectional view of a portion of the FIG. 1a RV as mounted in a suitable specimen handling device.
**FIG. *1c*** is an enlarged perspective cross-sectional partial view of a different RV incorporating various aspects of the invention.
**FIG. *2*** is a schematic of the fluid delivery components for aspirating and dispensing metered amounts of liquids in conjunction with the present invention.
**FIG. *2a*** is a schematic diagram of a valve usable in the system of FIG. 2.
**FIG**. ***3*** is a schematic of the electronic control details of the device in the invention.
**FIG. *4*** is a perspective view of a specimen handling device and showing one RV according to the present invention.

### DETAILED DESCRIPTION OF THE INVENTION

The present invention addresses handling of a liquid sample, particularly relating to the extraction of specific analytes present in the sample. The invention is useful in cost saving resulting from reduction of the number of disposable pipette tips, reagent economy, and time saving. In one aspect of the present invention, an RV with an open top and a drain opening at or near its bottom is described. RVs according to the present invention will be generically identified herein by reference numeral 10, with specific RVs shown in the figures identified by reference numerals 10a and 10d. The drain opening is normally closed by the presence of a valve, and the valve is capable of being actuated to open by the application of pressure differential between the outside of the bottom opening and the liquid inside the vessel.

For simplifying device construction, ease of use, and cost saving in the present invention, the valve may be advantageously attached to the bottom of the RV. This may be accomplished using grooves or other support features designed specifically to support the valve's geometry. When no pressure differential is applied, the valve will close the drain opening and the valve also will advantageously re-close after the pressure application ceases because extracting an analyte from a sample normally requires multiple fluid exchanges.

The valve may suitably include a bottom portion which is designed to match the geometry of the drain opening at the bottom of the RV so that it covers the opening, with a slit in the bottom portion. The valve material should be compatible with the fluid(s) to be processed (e.g., may be inert with regard to interaction with reagents utilized in nucleic acid extraction), and advantageously may be elastomeric, and/or possess elastomeric properties such that the slit is normally closed, with the bottom portion flexing to open the slit upon application of selected relative pressure and the slit closing after the selected relative pressure ceases. Examples of suitable elastomeric polymers may include, but are not limited to, polyisoprenes, natural and vulcanized rubber, polybutadiene, butyl rubbers, nitrile rubber (buna N rubbers) and silicone rubber.

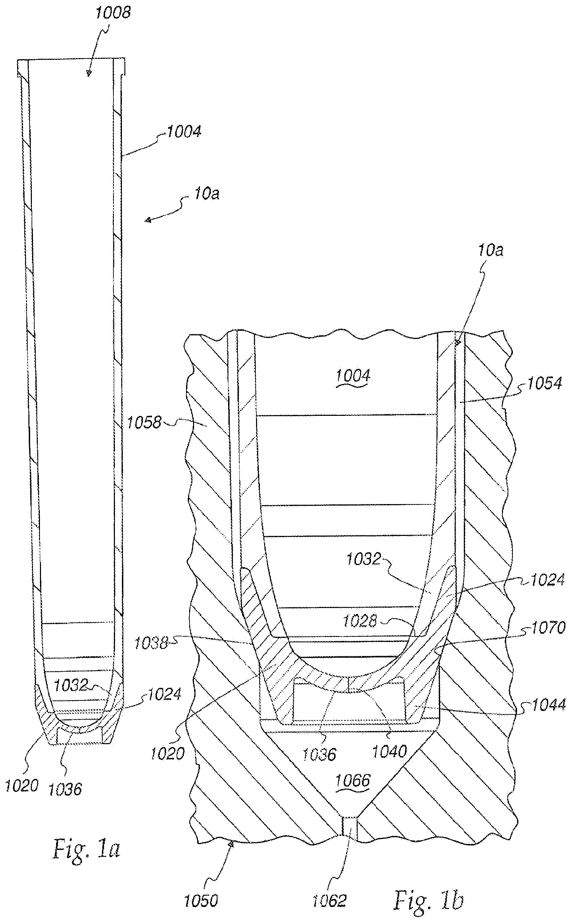

Various valve configurations would be suitable for use with the present invention, with a variety of such valves shown in **FIGS. 1a-1c** below.

**FIGS. 1a-1b** show an RV 10a which includes a suitable generally cylindrical body 1004 defined by vertically extending closing walls defining the sides of the containment space of the RV 10a (the cylindrical body 1004 may, for example, advantageously be slightly tapered along its length to facilitate placement in a cylindrical opening without binding therein as described below). The top 1008 of the RV 10a is open to allow fluid with analyte to be added to the RV 10a, and also to allow atmospheric pressure to be present at the top of any such fluid.

An elastic valve 1020 is suitably secured to the bottom of the cylindrical body 1004. Advantageously, the elastic valve 1020 may include a conical thinned rim 1024 around its upper portion, with a shoulder 1028 defined beneath the rim 1024, whereby a cooperating conical thinned bottom portion 1032 of the cylindrical body 1004 is received in, and suitably secured to, the cylindrical body rim 1024. The elastic valve 1020 also includes a conically tapered outer surface 1038.

Advantageously, the material of both the cylindrical body 1004 and the elastic valve 1020 may be inert with regard to interaction with reagents utilized in nucleic acid extraction.

The bottom, center portion 1036 of the elastic valve 1020, closing the bottom of the containment space, may be flat or concave and, advantageously, may be semispherical. A slit 1040 is advantageously provided at the lowermost (center) portion of the bottom, center portion 1036 of the elastic valve 1020.

The slit 1040 has a closed condition blocking fluid from passing through the valve 1020 and an open position defining a drain opening allowing fluid to pass through the slit 1040. More specifically, the elastic valve 1020 is strong enough to support a head of fluid in the containment space of the RV 10a with the slit 1040 in the closed condition, and yet elastically flexible enough to be elastically deformed (e.g., drawn down) to place the slit 1040 in the open position responsive to a selected differential between the pressure at the RV top 1008 and a lower pressure beneath the elastic valve 1020.

The elastic valve 1020 also includes a lip 1044 extending downwardly beyond and around the concave bottom, center portion 1036. The lip 1044 provides stability to the elastic valve 1020 to assist in maintaining the slit 1040 in its closed position, and then allowing the slit 1040 to transition to its open position for desired draining without undesirable leaking between positions.

One specific design which has been found to be suitable includes a cylindrical body 1004 made of polypropylene and having an inner diameter of about 0.927 cm (about .356 inch) and a 20 degree taper extending about about 0.317 cm (1/8 inch) height defining the thinned bottom portion 1032. The elastic valve 1020 is made of GLS 800-B30 TPE, such as may be purchased from GLS Corporation, 833 Redgeview Drive, McHenry, IL, U.S.A., and has a height of about 0.686 cm (about 0.27 inch) and a bottom, center portion 1036 with a radius of about 0.396 cm (about 0.156 inch) and a thickness of about 0.066 cm (about 0.026 inch), with the valve downwardly extending lip 1044 having a height of about 0.190 cm (about 0.075) inch and an inner surface with a radius of about 0.571 cm (about 0.225 inch). The slit 1040 is a substantially straight cut in the bottom of the center portion 1036 and has a horizontal length of about 0.254 cm (about 0.10 inch).

As illustrated in **FIG. 1b**, a RV 10a such as shown in **FIG. 1a** is mounted in a suitable carrier 1050. Though only one cylindrical opening 1054 for mounting one RV 10a is shown, it should be appreciated that the carrier 1050 may, and typically will be, a block 1058 with multiple openings 1054 allowing an array of multiple RVs 10a to be mounted therein, where the block 1058 will define and occupy the space separating the openings 1054 from one another.

The block 1058 also includes passages 1062 through the block 1058 which connect the area 1066 beneath each mounted RV 10a to a suitable vacuum source (not shown in **FIG. 1b**). Further, it should be appreciated that the bottom of the carrier openings 1054 in the carrier 1050 may include a reduced diameter portion 1070 which the RV elastic valve 1020 seats against to both support the RV 10a and to provide a seal separating the area 1066 beneath the reduced diameter portion 1070 from the space between the RV 10a and the cylindrical opening 1054 above the reduced diameter portion 1070, to thereby facilitate the reduction in pressure in the area 1066 when a vacuum is applied to the passages 1062.

It should thus be appreciated that an RV 10a which is located in an opening 1054 in the carrier 1050 can support a head of fluid in the RV 10a as previously described. When a sufficient vacuum is applied via the passages 1062 to the area 1066 beneath the RV 10a, the elastic valve 1020 will be elastically deformed (e.g., drawn down) as a result of both the head of fluid above the valve 1020 and the pressure differential (i.e., the differential between atmospheric pressure on top of the fluid and the vacuum beneath the elastic valve 1020) to place the slit 1040 in the open position whereby the fluid will be drawn through the open slit and down through the passages 1062 to remove the fluid from the RV 10a as desired. It should be appreciated, of course, that rather than drawing a vacuum beneath the valve 1020, an increased pressure could be applied to the top of the fluid, to accomplish the same result, though the described use of vacuum is mechanically easier to use for this purpose.

Thus, it should be appreciated that the RV 10a and carrier 1050 as described above can be used to extract an analyte in a fluid as follows. Specifically, as is well known in the art, liquid reagents for binding the analyte to magnetic microparticles may be delivered to the RV 10a, and then a magnetic field may be applied to immobilize the magnetic particles. With the magnetic particles immobilized, the selected pressure differential may be applied to open the valve slit 1040 and selectively drain fluid through slit 1040 and the area 1066 beneath the RV 10a and the passages 1062.

Thereafter, liquid reagents may be delivered to wash the magnetic particles, and the differential pressure may again be applied (e.g., by application of a vacuum in the carrier passages 1062) to selectively drain that fluid through the RV valve slit 1040.

Liquid reagents may then be delivered to the RV 10a to elute the analyte from the magnetic microparticles.

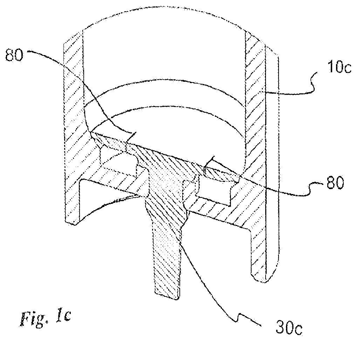

**FIG. 1c** illustrates another embodiment of an RV 10c, wherein an actuatable, elastomeric valve 30c is fitted to the inside of the RV. The valve in this embodiment contains slits, 80, which are normally closed in the absence of differential pressure. However, upon application of differential pressure or vacuum to the outside bottom of the valve, the slits deform, leading to the formation of an opening and allowing the fluid in the sample to flow through the drain opening.

The vessels and valves are particularly advantageous inasmuch as such vessels are low cost, minimize the use of disposables, e.g., pipette tips and allow more efficient processing. The manner of draining fluid 20 from the RVs 10 should thus be appreciated to be fast and convenient. Further, RVs 10 according to the present invention advantageously avoid leakage and contamination and also provide ease and reproducibility in manufacturing. Moreover, it should be understood that aspects of the present invention encompass still further vessels and valves that can be deformed by the application of, for example, vacuum and should be familiar to those skilled in the art.

An array of the RVs thus described according to the present invention may be supported on a carrier that contains RV receptacles matching the contour of the RVs. The RV receptacles contain connecting fluid passageways which lead to a common drainage passageway and to a discharge opening. Advantageously, the drainage passageway is of sufficient volume as to accommodate common draining and avoid cross contamination among RVs resulting from fluid drained from one vessel passing along the passageway and contacting the valve of another RV in the carrier. A source of applying a pressure differential (e.g., a vacuum pump) may be connected to the discharge opening. Liquid waste will accumulate in the waste containers where waste container acts as a trap. A valve may selectively connect the discharge opening to a vacuum source (waste container) to selectively initiate and terminate the vacuum.

Typically, the drain opening permits draining of the fluid only when the relative pressure on opposite sides of the valve is at least a selected amount. Thus, the selected amount of relative pressure will depend on both the pressure resulting from the head of fluid as well as the relative pressure between the top of the fluid and underside of the valve (*i.e.*, the relative pressure on opposite sides of the valve). If variations in the amount of fluid to be included in the RV are expected, the valve should advantageously be configured to be able to at least support the maximum head of fluid expected, and the vacuum source should be able to supply a sufficient vacuum to open the valve even if the minimum fluid head expected is present. Alternatively, it should be appreciated that the relative pressure may be supplied by adding pressure to the top of the RV instead of, or in combination with, a vacuum applied to the underside of the valve.

As is known in the art, pairs of bar magnets may be included in the vicinity of each RV, with the magnets being each independently movable, primarily in the vertical direction. Other magnets, such as electromagnets may be used within the scope of the present invention, with the magnets used to translate the magnetic microparticles before and/or after the magnetic microparticles bind the analyte of interest. The magnets' motion thus described may also be used to mix the microparticles, hence the content of the RV. For example, analytes in fluids in RVs may be processed by (1) adding a sample containing the analyte of interest to an RV according to the present invention, (2) binding the analyte to a magnetic particle, (3) using the magnets to introduce a magnetic field into the vessel which draws the magnetic particle and bound analyte to the side of the vessel, (4) moving the magnets vertically along the height of the RV to draw the magnetic particle and bound analyte together into a pellet in the RV, and (5) applying differential pressure between the top of the fluid and the bottom of the drain opening sufficient to open the valve and drain the fluid through the drain opening.

Multiple carriers may be arranged to support RVs in a repeating pattern, consisting of rows, each of preferably eight RVs repeated up to twelve times, whereby a total of ninety-six RVs may be processed in a 'batch'.

The magnets may be shielded by magnetic shielding elements which may be composed of materials known in the art, such as a mu-metal.

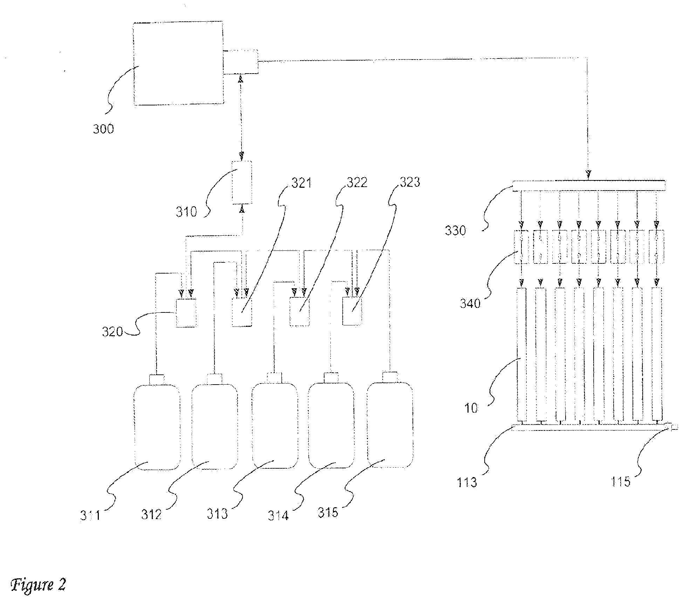

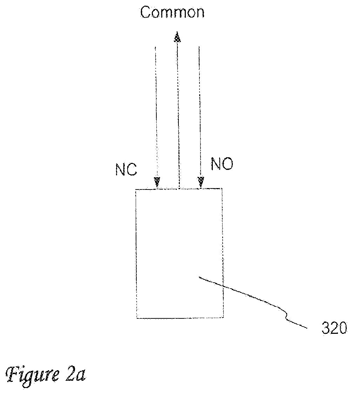

Fluid delivery components for aspirating and dispensing metered amounts of liquids in conjunction with the present invention are schematically illustrated in FIG. 2. For example, a pump, 300, such as the Digispense 2000, which can be obtained from IVEK Corporation, of North Springfield, VT, USA (http:f/www.ivek.com/digispense2000.cont.mod.html), may be used to aspirate certain amounts of liquids from bulk liquid containers, 311-315, which contain the reagents necessary for the extraction of specific analytes, such as nucleic acids. For example, the containers 311-315 may contain bulk supplies of lysis buffer, washes, and elution buffers. It should be noted that the number of such containers depends on the specific application for the present invention, and that the 5-reagent example in **FIG. 2** is only an illustration. Several valves 320-323 which may be controlled electronically are used to control the liquid flow, with the valve connections arranged to minimize dissimilar fluid mixing and minimize wash flushing between aspirations. The valves (*e.g*., 320 in **FIG. 2a**) may possess three ports: NO: Normally Open, NC: Normally Close, and Common. Valves with different port configurations may also be used, and should be familiar to those skilled in the art. The pump 300 dispenses specific fluids to the RV arrays through a dispense arm 330. Additional valves 340 may be utilized to control dispensing to particular RVs.

The bulk liquid containers 311-315 may be connected to a traveling liquid-dispense nozzle and flexible tubing (not shown).

After the wash fluid has been discharged into all of the selected RVs 10, the valves 320-323 can be instructed to supply a different fluid if needed based on the testing being accomplished. For example, if lysis buffer is to be dispensed, valve 320 may be instructed to open the port to the reagent container 310, where the pump 300 aspirates, for example, 2 mL of the lysis buffer. The port to the reagent bottle is then closed, the port to the RV 10 is opened, and the pump 300 dispenses the aspirated fluid into the RV. It should be understood by those familiar with the art that alternate dispense schemes could be used with the invention.

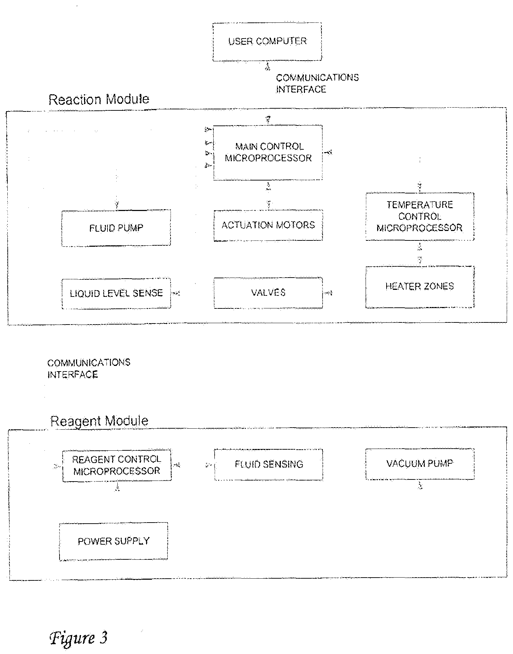

**FIG. 3** is a schematic of control details which may be used with the invention. A main control processor sends instructions and receives state signals from the electromechanical controls of the elements comprising the invention: temperature control microprocessor unit, valve actuation, motor actuation and motion control, fluid pump, liquid level sense, and the reagent control microprocessor. Multiple auxiliary control microprocessor units may control various elements of the device (e.g., the temperature microprocessor unit controls the heating zones of the RVs). A microprocessor may control the bulk reagents, sensing fluid levels and reporting their states to the main microprocessor. The main control processor may be controlled by a personal computer with the appropriate graphic/text interface with instructions for the user and report generation capabilities of the relevant system state values. It should be noted that other control configurations and combinations are feasible, and that those skilled in the art may utilize different control mechanisms.

Devices based on the above-described elements in the invention may be constructed for the purpose of extracting an analyte from a liquid sample. In addition to the above elements, features may be included to facilitate the processing of extracting an analyte from a specimen, such as heating elements, liquid metering and dispensing devices, and liquid level sense circuitry.

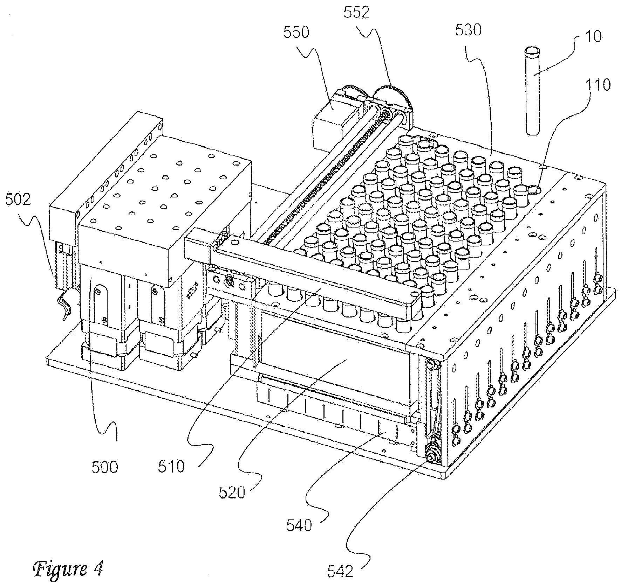

**FIG. 4** shows an example of such a device, which encompasses a 96-sample batch processing capability. The device contains liquid metering pumps 500 connected by appropriate tubing (not shown) to a dispense arm 510 which contains 8 nozzles spaced to align with the top openings of eight RVs. A dispense arm translation motion mechanism having an x-y direction above the RVs, for example, a motor 550 and gears 552, is used to translate the dispense arm to the different rows of RVs 10. The device also includes magnet bars 540 and heating elements 520 such as previously described with **FIG. 2****.** The fluidics channels from the bottom of the drainage passageways are connected to a common discharge connector 542, with an appropriate vacuum source (not shown in **FIG. 4**) connected to the common fluid discharge line 542. Other components of the device (not shown) should be familiar to those skilled in the art and may include, for example, controls for motion translation, vacuum pump activation, heating, magnet translation, liquid dispense and liquid level sense.

Although the present invention as described may be used as a stand-alone, it may also be used with a suitable automated testing instrument as a platform to facilitate analyte extraction and testing. Since many features of such instruments, though usable with the present invention, do not form a part of the invention, they are therefore not shown in the figures. Those skilled in the art would find many examples of, for example, robotic arms and fluid dispensing components available in the marketplace (e.g., from Tecan, AG). The use of an automated testing platform may advantageously also include one or more of the following features: (1) a receptacle to hold and segregate used pipette tips from samples and reagents such that contamination from used tips is minimized, (2) aerosol control devices, for example without limitation, (a) a sample tube or reagent tube sealer, (b) electrodes for treating surfaces and/or liquids with electrical current capable of modifying nucleic acids, (c) an ultraviolet light source capable of degrading or modifying nucleic acids, (d) an apparatus for causing laminar air flow in or around the automatic testing device as an additional protection against contamination, and (3) an optical detector (*e.g*., a fluorometer) for measuring the light absorbance or fluorescence of processed samples. Those skilled in the art who obtain an understanding of the present invention will recognize such features, such as disclosed, for example, in U.S. Patent No. 7,125,722 titled "Apparatus and Method for Handling Fluids for Analysis", U.S. Pat. No. 6,413,780, titled "Structure and Method for Performing a Determination of an Item of Interest in a Sample", and U.S. Publication No. 2002-0127727 also titled "Structure and Method for Performing a Determination of an Item of Interest in a Sample".

Particular interest is given to the case where the extraction of the analyte is accomplished by binding of the analyte to specific magnetic microparticles, for example, those that can be obtained from Promega Co., of Madison, Wisconsin, IL, USA. When magnetic microparticles are used, a magnetic field is applied to immobilize and gather (capture) the magnetic microparticles in a certain region of the RV, where the rest of the fluid sample may be discarded by the application of vacuum to open the drain opening.

Of particular interest is the application of aspects of the present invention to the extraction of nucleic acid (DNA and RNA) from a biological sample, and particularly using the magnetic microparticles mentioned above. An example achieving such extraction using a device of this invention is given in the Example below.

### Example

The example demonstrates the benefits of features of the invention (*e.g*., the fluid drainage and re-closure of the actuatable valves of the invention) in comparison to established procedures of nucleic acid extraction. In practice the reaction vessels of the invention (RVs) are supported on a single carrier board (SCB) as shown in **FIG. 2****,** with a vacuum pump, heating elements and temperature controller such as may be obtained from Watlow Electric Manufacturing Company, St. Louis, Missouri, USA. Liquid reagents were dispensed and aspirated using the m2000sp™ instrument, which is an automated fluid handling system which can be obtained from Abbott Molecular Inc., Des Plaines, IL, USA, as outlined below. The RV depicted in **FIG. 1b** was used in the example.

### Extraction of HCV RNA from Plasma Samples

Plasma samples spiked with Hepatitis C Virus (HCV) were used to simulate "unknown" plasma samples. All samples were made using the Low Positive Control at 427 International Unit per milliliter, IU/mL available from Abbott Molecular.

96 RVs were used, and labeled A1 through H12. An internal control was added to each 'sample' to ensure the validity of the run. Reagents used for the extraction are also obtainable from Abbott Molecular in kits, or can be formulated by those skilled in the art. For this example, these reagents include HCV internal control #8100/4J86Y, HCV assay Enzyme #566850099, HCV oligonucleotide mix #4J86 L0090, Activator #93591099, and Sample Preparation System kit 02K02-24, which includes the sample preparation reagents for lysis (mLysis), Wash1 (mWash1), Wash2 (mWash2), microparticles (mMicroparticles), and elution (mElution).

RNA was extracted from the samples according to the following procedure:
1- Set the temperature of the Reaction Vessel (RV) to 50C.
2- Transfer 0.1 milliliter (mL) of magnetic microparticles to RV.
3- Transfer 2.4 mL Lysis Buffer to RV.
4- Transfer 0.025 mL assay specific Internal Control to RV.
5- Transfer 0.5 mL Sample containing the target to be analyzed to RV and Mix.
6- Incubate mixture for 1200 seconds.
7- Set RV incubation temperature to room temperature.
8- Immobilize micro particles by positioning movable magnets (capture position).
9- Remove liquid waste from RV by applying vacuum (opens the RV valve).
10-Transfer 0.750 mL RNA Wash 1 to RV.
11-Perform RV mix with magnets.
12-Immobilize the magnetic microparticles by positioning movable magnets (capture position).
13-Remove liquid waste from RV by applying vacuum.
14-Repeat steps 10-13 for a second wash 1.
15-Transfer 2.5mL RNA Wash 2 to RV.
16-Repeat steps 11-13.
17-Transfer 0.850 mL RNA Wash 2 to RV.
18-Repeat steps 11-13.
19-Transfer 0.025 mL RNA phosphate buffer to RV.
20-Set RV Incubation Temperature to 75C.
21-Incubate RV 600 Seconds.
22-Perform RV Mix with Magnets.
23-Incubate RV 600 Seconds.
24-Transfer 0.063 mL RNA Wash 2 to RV.
25-Set RV incubation temperature to room temperature.
26-Capture particles and transfer eluate (purified nucleic acids) from RV to amplification vessel.
   Following extraction, the RNA was reverse transcribed to DNA. The DNA was amplified by PCR and detected. Reagents used for reverse transcription and PCR amplification are also obtainable from Abbott Molecular in kits, or can be formulated by those skilled in the art. The reverse transcription and PCR amplification were performed according to the following procedure using the m2000rt instrument, also available from Abbot Molecular.
27-Initialize the m2000rt instrument and set up reaction plate specifications.
28-Thaw PCR amplification reagents.
29-Transfer 971 microliters of oligonucleotide mix to each PCR enzyme bottle, mix by pipetting approximately 4 times.
30-Transfer 249 microliters of activator to each enzyme bottle, mix by pipetting approximately 4 times.
31-Pool the 4 bottles of mastermix into a single tube.
32-Transfer 50 microliters of mastermix into wells of amplification tray.
33-Transfer 50 microliters of each sample into wells of amplification tray.
34-Seal tray and load into m2000rt.
35-Run protocol "m2000 0.5 ml HCV RNA Application version 3.0".
36-After run has completed, download data and analyze.

### Results

The amount of amplified DNA obtained was substantially equivalent to that obtained using conventional reaction vessels. The advantages of using the invention are faster turn around time, cost reduction, and less solid waste.

## Claims

1. A reaction vessel (10) for the extraction of an analyte in a fluid, said reaction vessel (10) having:
i. a cylindrical body (1004) including a fluid containment space defined by substantially vertical walls, the cylindrical body (1004) including a conical thinned bottom portion (1032);
ii. an opening at the top (1008) of said vertical walls; and
iii. an elastic valve (1020) secured to the vertical walls to define the bottom of said containment space, the elastic valve (1020) including a conical thinned rim (1024) around its upper portion and a shoulder (1028) beneath the conical thinned rim (1024), the conical thinned bottom portion (1032) of the cylindrical body (1004) is received in the conical thinned rim (1024) such that a bottom of said vertical walls abut said shoulder (1028),
said elastic valve (1020) being concave and semispherical at the center (1036) of the containment space, said elastic valve (1020) having a slit (1040) positioned at a lowermost portion of the bottom of the containment space and being through the bottom of the concave center portion (1036) of the elastic valve (1020), said slit (1040) having a closed condition blocking fluid from passing through said slit (1040) and an open position defining a drain opening allowing fluid to pass through said slit (1040), and
said elastic valve (1020) adapted to
support a quantity of fluid in said containment space of said reaction vessel (10) with said slit (1040) in said closed condition, and
be elastically deformed and drawn down to force said slit (1040) in said open position responsive to a selected differential between a first pressure at the top opening (1008) and a second pressure beneath the elastic valve (1020), wherein the first pressure is greater than the second pressure,
wherein said elastic valve (1020) is constructed of a material possessing elastomeric properties such that said slit (1040) is normally in said closed condition, said lowermost portion of said bottom of said containment space flexing to open said slit (1040) to said open position upon application of said selected differential and said slit (1040) closing after the selected differential ceases.

2. The reaction vessel (10) of claim 1, further comprising a lip (1044) extending downwardly beyond and around the concave bottom of the elastic valve (1020).

3. The reaction vessel (10) of claim 1, wherein said concave bottom of said elastic valve (1020) is semispherical.

4. The reaction vessel (10) of claim 1, wherein said elastic valve (1020) is adapted to elastically deform to force said slit (1040) in said open position when said first pressure is atmospheric pressure and said second pressure is a partial vacuum.

5. The reaction vessel (10) of claim 1, wherein said vertical walls are substantially cylindrical with the bottom portion tapered conically inwardly, and said conically tapered bottom portion is secured in an upper tapered portion of said elastic valve.

6. The reaction vessel (10) of claim 1, wherein said valve (1020) is a material which is inert with regard to interaction with reagents utilized in nucleic acid extraction.

7. A device (1050) for the extraction of an analyte in a fluid, comprising:
a. at least one reaction vessel (10) as defined in claim 1 for testing of an analyte in a fluid;
b. a support (1050) for the reaction vessel (10) that contains passages (1054) adapted to communicate with the bottom drain openings (1062) of reaction vessels (10); and
c. a source of differential pressure adapted to selectively drain fluid through the drain openings (1062) in supported reaction vessels (10).

8. The device of claim 7, wherein said source of differential pressure is a vacuum source for drawing a vacuum in said passages.

9. The device of claim 7, wherein said elastic valve (1020) includes a lip (1044) extending downwardly beyond and around the valve slit (1040) to protect said slit (1040).

10. The device of claim 9, wherein the reaction vessel (10) vertically extending walls are substantially cylindrical and the reaction vessel support for each reaction vessel includes a reduced diameter portion (1066) on which the vessel elastic valve (1020) seats to support the reaction vessel (10) in said support (1050).

11. A method for extracting an analyte in a fluid using reaction vessels (10) according to claim 1, comprising:
a. supporting the reaction vessel (10) on a support that contains passages adapted to communicate with the bottom drain openings (1062) of reaction vessels (10);
b. delivering liquid reagents for binding said analyte to magnetic microparticles;
c. applying a magnetic field to immobilize the magnetic particles;
d. applying the selected pressure differential to open the valve slit (1040) and selectively drain fluid through the drain openings (1062) in supported reaction vessels (10);
e. delivering liquid reagents to wash the magnetic particles followed by applying differential pressure adapted to selectively drain fluid through the drain openings (1062) in supported reaction vessels (10); and
f. delivering liquid reagents to elute the analyte from the magnetic microparticles.

12. The method of claim 11, wherein said applying differential pressure steps comprise selectively creating a vacuum beneath said drain opening (1062).

## Patentansprüche

1. Ein Reaktionsgefäß (10) für die Extraktion eines Analyten in einem Fluid, wobei das Reaktionsgefäß (10) Folgendes aufweist:
i. einen zylindrischen Körper (1004) einschließlich einem Fluideinschlussraum definiert durch im Wesentlichen vertikale Wände, wobei der zylindrische Körper (1004) einen konisch verdünnten Bodenabschnitt (1032) einschließt;
ii. eine Öffnung oben (1008) an den genannten vertikalen Wänden; und
iii. ein elastisches Ventil (1020) befestigt an den vertikalen Wänden, um den Boden des genannten Einschlussraums zu definieren, wobei das elastische Ventil (1020) einen konisch verdünnten Rand (1024) um seinen oberen Teil herum und eine Schulter (1028) unter dem konisch verdünnten Rand (1024) einschließt, der konisch verdünnte Bodenabschnitt (1032) des zylindrischen Körpers (1004) wird so in dem konisch verdünnten Rand (1024) aufgenommen, dass ein Boden der genannten vertikalen Wände an die genannte Schulter (1028) angrenzt,
wobei das genannte elastische Ventil (1020) konkav und halbkugelförmig in der Mitte (1036) des Einschlussraums ist, wobei das elastische Ventil (1020) einen Schlitz (1040) positioniert am untersten Abschnitt des Bodens des Einschlussraums hat und durch den Boden des konkaven Mittelabschnitts (1036) des elastischen Ventils (1020) geht, wobei der genannte Schlitz (1040) einen geschlossenen Zustand hat, der Fluid daran hindert durch den genannten Schlitz (1040) hindurch zu treten, und eine offene Position, die eine Ausgussöffnung definiert, die dem Fluid erlaubt durch den Schlitz (1040) hindurch zu treten, und
wobei das genannte elastische Ventil (1020) angepasst ist, um eine Menge an Fluid in dem genannten Einschlussraum des genannten Reaktionsgefäßes (10) mit dem genannten Schlitz (1040) in dem genannten geschlossenen Zustand zu stützen, und
elastisch deformiert und heruntergezogen zu werden, um den genannten Schlitz (1040) in die genannte offene Position zu zwingen als Reaktion auf ein gewähltes Differential zwischen einem ersten Druck an der oberen Öffnung (1008) und einem zweiten Druck unter dem elastischen Ventil (1020), worin der erste Druck größer ist als der zweite Druck,
worin das genannte elastische Ventil (1020) aus einem Material hergestellt ist, das elastomere Eigenschaften besitzt, so dass der genannte Schlitz (1040) normalerweise in dem geschlossenen Zustand ist, wobei sich der unterste Abschnitt des genannten Bodens des genannten Einschlussraums biegt, um den Schlitz (1040) nach Anbringung des genannten gewählten Differentials in die genannte offene Position zu öffnen, und der genannte Schlitz (1040) sich schließt nachdem das gewählte Differential nachlässt.

2. Das Reaktionsgefäß (10) gemäß Anspruch 1, weiter umfassend eine Lippe (1044), die sich abwärts außerhalb und um den konkaven Boden des elastischen Ventils (1020) herum erstreckt.

3. Das Reaktionsgefäß (10) gemäß Anspruch 1, worin der genannte konkave Boden des genannten elastischen Ventils (1020) halbkugelförmig ist.

4. Das Reaktionsgefäß (10) gemäß Anspruch 1, worin das genannte elastische Ventil (1020) angepasst ist sich elastisch zu deformieren, um den genannten Schlitz (1040) in die genannte offene Position zu zwingen, wenn der genannte erste Druck Umgebungsdruck ist und der genannte zweite Druck ein Teilvakuum ist.

5. Das Reaktionsgefäß (10) gemäß Anspruch 1, worin die genannten vertikalen Wände im Wesentlichen zylindrisch sind mit dem Bodenabschnitt konisch nach innen verjüngt, und der genannte konisch verjüngte Bodenabschnitt ist in einem oberen verjüngten Abschnitt des genannten elastischen Ventils fixiert.

6. Das Reaktionsgefäß (10) gemäß Anspruch 1, worin das genannte Ventil (1020) ein Material ist, welches hinsichtlich einer Wechselwirkung mit Reagenzien, die in der Nukleinsäureextraktion verwendet werden, inert ist.

7. Eine Vorrichtung (1050) für die Extraktion eines Analyten in einem Fluid, die Folgendes umfasst:
a. mindestens ein Reaktionsgefäß (10) wie in Anspruch 1 definiert zum Testen eines Analyten in einem Fluid;
b. einen Träger (1050) für das Reaktionsgefäß (10), der Durchgänge (1054) enthält, die angepasst sind, um mit den Bodenausgussöffnungen (1062) der Reaktionsgefäße (10) zu kommunizieren; und
c. eine Quelle von Differentialdruck, die angepasst ist, um selektiv Fluid durch die Ausgussöffnungen (1062) in gestützen Reaktionsgefäßen (10) abzulassen.

8. Die Vorrichtung gemäß Anspruch 7, worin die genannte Quelle von Differentialdruck eine Vakuumquelle zum Erzeugen eines Vakuums in den genannten Durchgängen ist.

9. Die Vorrichtung gemäß Anspruch 7, worin das genannte elastische Ventil (1020) eine Lippe (1044) einschließt, die sich abwärts außerhalb und um den Ventilschlitz (1040) herum erstreckt, um den genannten Schlitz (1040) zu schützen.

10. Die Vorrichtung gemäß Anspruch 9, worin die sich vertikal erstreckenden Wände des Reaktionsgefäßes (10) im Wesentlichen zylindrisch sind, und der Reaktionsgefäßträger für jedes Reaktionsgefäß schließt einen Abschnitt (1066) mit verkleinertem Durchmesser ein, auf welchem das elastische Ventil (1020) des Gefäßes sitzt, um das Reaktionsgefäß (10) in dem genannten Träger (1050) zu stützen.

11. Ein Verfahren zum Extrahieren eines Analyten in einem Fluid unter Verwendung von Reaktionsgefäßen (10) gemäß Anspruch 1, das Folgendes umfasst:
a. Stützen des Reaktionsgefäßes (10) auf einem Träger, der Durchgänge enthält, die angepasst sind, um mit den Bodenausgussöffnungen (1062) der Reaktionsgefäße (10) zu kommunizieren;
b. Zuführen von flüssigen Reagenzien zum Binden des genannten Analyten an magnetische Mikropartikel;
c. Anlegen eines Magnetfeldes, um die magnetischen Partikel zu immobilisieren;
d. Anbringen des gewählten Druckdifferentials, um den Ventilschlitz (1040) zu öffnen und selektiv Fluid durch die Ausgussöffnungen (1062) in gestützten Reaktionsgefäßen (10) auszulassen;
e. Zuführen von flüssigen Reagenzien, um die magnetischen Partikel zu waschen, gefolgt von Anlegen eines Differentialdrucks, der angepasst ist, um selektiv Fluid durch die Ausgussöffnungen (1062) in gestützten Reaktionsgefäßen (10) auszulassen; und
f. Zuführen von flüssigen Reagenzien, um den Analyten aus den magnetischen Mikropartikeln zu eluieren.

12. Das Verfahren gemäß Anspruch 11, worin die genannten Schritte des Anlegens von Differentialdruck das selektive Erzeugen eines Vakuums unterhalb der genannten Ausgussöffnung (1062) umfassen.

## Revendications

1. Cuve de réaction (10) pour l'extraction d'un analyte dans un fluide, ladite cuve de réaction (10) présentant :
i. un corps cylindrique (1004) incluant un espace de confinement de fluide défini par des parois essentiellement verticales, le corps cylindrique (1004) incluant une partie inférieure affinée conique (1032) ;
ii. un orifice au sommet (1008) desdites parois verticales ; et
iii. une valve élastique (1020) fixée contre les parois verticales pour définir le fond dudit espace de confinement, la valve élastique (1020) incluant un rebord affiné conique (1024) autour de sa partie supérieure et un épaulement (1028) sous le rebord affiné conique (1024), la partie inférieure affinée conique (1032) du corps cylindrique (1004) est reçue dans le rebord affiné conique (1024) de telle sorte qu'un fond desdites parois verticales vienne en butée contre ledit épaulement (1028),
ladite valve élastique (1020) étant concave et hémisphérique au centre (1036) de l'espace de confinement, ladite valve élastique (1020) présentant une fente (1040) positionnée au niveau d'une partie la plus basse du fond de l'espace de confinement et étant au travers du fond de la partie de centre (1036) concave de la valve élastique (1020), ladite fente (1040) présentant un état fermé empêchant un fluide de passer à travers ladite fente (1040) et une position ouverte définissant un orifice d'écoulement permettant à un fluide de passer à travers ladite fente (1040), et
ladite valve élastique (1020) conçue pour
supporter une quantité de fluide dans ledit espace de confinement de ladite cuve de réaction (10) avec ladite fente (1040) dans ledit état fermé, et
être élastiquement déformée et tirée vers le bas pour forcer ladite fente (1040) dans ladite position ouverte en réponse à un différentiel sélectionné entre une première pression au niveau de l'orifice au sommet (1008) et une seconde pression sous la valve élastique (1020), dans laquelle la première pression est supérieure à la seconde pression,
dans laquelle ladite valve élastique (1020) est faite d'un matériau possédant des propriétés élastomères telles que ladite fente (1040) soit normalement dans ledit état fermé, ladite partie la plus basse dudit fond dudit espace de confinement fléchissant pour ouvrir ladite fente (1040) vers ladite position ouverte en cas d'application dudit différentiel sélectionné et ladite fente (1040) se fermant dès lors que cesse le différentiel sélectionné.

2. Cuve de réaction (10) selon la revendication 1, comprenant en outre une lèvre (1044) s'étendant vers le bas sous le et autour du fond concave de la valve élastique (1020).

3. Cuve de réaction (10) selon la revendication 1, dans laquelle ledit fond concave de ladite valve élastique (1020) est hémisphérique.

4. Cuve de réaction (10) selon la revendication 1, dans laquelle ladite valve élastique (1020) est conçue pour se déformer élastiquement afin de forcer ladite fente (1040) dans ladite position ouverte lorsque ladite première pression est une pression atmosphérique et ladite seconde pression est un vide partiel.

5. Cuve de réaction (10) selon la revendication 1, dans laquelle lesdites parois verticales sont essentiellement cylindriques avec la partie inférieure effilée de manière conique vers l'intérieur, et ladite partie inférieure effilée de manière conique est fixée dans une partie supérieure effilée de ladite valve élastique.

6. Cuve de réaction (10) selon la revendication 1, dans laquelle ladite valve (1020) est un matériau qui est inerte en ce qui concerne une interaction avec des réactifs utilisés dans une extraction d'acide nucléique.

7. Dispositif (1050) pour l'extraction d'un analyte dans un fluide, comprenant :
a. au moins une cuve de réaction (10) selon la revendication 1 pour tester un analyte dans un fluide ;
b. un support (1050) pour la cuve de réaction (10) qui contient des passages (1054) conçus pour communiquer avec les orifices d'écoulement (1062) inférieurs de cuves de réaction (10) ; et
c. une source de pression différentielle conçue pour canaliser de manière sélective un fluide à travers les orifices d'écoulement (1062) dans des cuves de réaction (10) bénéficiant d'un support.

8. Dispositif selon la revendication 7, dans lequel ladite source de pression différentielle est une source de vide pour créer un vide dans lesdits passages.

9. Dispositif selon la revendication 7, dans lequel ladite valve élastique (1020) inclut une lèvre (1044) s'étendant vers le bas sous le et autour de la fente (1040) de valve pour protéger ladite fente (1040).

10. Dispositif selon la revendication 9, dans lequel les parois de cuve de réaction (10) s'étendant verticalement sont essentiellement cylindriques et le support de cuve de réaction pour chaque cuve de réaction inclut une partie de diamètre réduit (1066) sur lequel la valve élastique (1020) de cuve s'assoit pour soutenir la cuve de réaction (10) dans ledit support (1050).

11. Procédé destiné à extraire un analyte dans un fluide utilisant des cuves de réaction (10) selon la revendication 1, comprenant :
a. le fait de soutenir la cuve de réaction (10) sur un support qui contient des passages conçus pour communiquer avec les orifices d'écoulement (1062) inférieurs de cuves de réaction (10) ;
b. la fourniture de réactifs liquides pour lier ledit analyte à des microparticules magnétiques ;
c. l'application d'un champ magnétique pour immobiliser les particules magnétiques ;
d. l'application de la pression différentielle sélectionnée pour ouvrir la fente (1040) de valve et canaliser de manière sélective un fluide à travers les orifices d'écoulement (1062) dans des cuves de réaction (10) bénéficiant d'un support ;
e. la fourniture de réactifs liquides pour laver les particules magnétiques, suivie par l'application d'une pression différentielle conçue pour canaliser de manière sélective un fluide à travers les orifices d'écoulement (1062) dans des cuves de réaction (10) bénéficiant d'un support ; et
f. la fourniture de réactifs liquides pour éluer l'analyte des microparticules magnétiques.

12. Procédé selon la revendication 11, dans lequel lesdites étapes d'application d'une pression différentielle comprennent une création sélective d'un vide sous lesdits orifices d'écoulement (1062).
